# EUROPEAN PATENT SPECIFICATION

(11) **EP 4 186 516 B1**
(45) Date of publication and mention of the grant of the patent: **11.12.2024**
(21) Application number: 21845200.1
(22) Date of filing: 21.07.2021
(51) Int. Cl.: A61K 33/44, A61K 9/14, A61K 9/70, A61P 31/12

(54) **PHARMACEUTICAL COMPOSITION COMPRISING GRAPHENE QUANTUM DOTS FOR USE IN THE TREATMENT OF VIRAL DISEASES CAUSED BY ADENOVIRUS INFECTIONS**
PHARMAZEUTISCHE ZUSAMMENSETZUNG MIT GRAPHEN-QUANTENPUNKTEN ZUR VERWENDUNG BEI DER BEHANDLUNG VON DURCH ADENOVIRUSINFEKTIONEN VERURSACHTEN VIRALEN KRANKHEITEN
COMPOSITION PHARMACEUTIQUE COMPRENANT DES POINTS QUANTIQUES DE GRAPHÈNE POUR LE TRAITEMENT DE MALADIES VIRALES CAUSÉES PAR DES INFECTIONS À ADÉNOVIRUS

(30) Priority: 21.07.2020 KR 20200090617; 20.07.2021 KR 20210094911
(43) Date of publication of application: 31.05.2023
(73) Proprietor: Biographene Inc., Suwon-si, Gyeonggi-do 16229 (KR)
(72) Inventor: KIM, Donghoon, Seoul 08816 (KR); PARK, Jong Bo, Seoul 08539 (KR)
(74) Representative: Dehns
(86) International application number: PCT/KR2021/009392
(87) International publication number: WO 2022/019640

(56) References cited:
- WO-A1-2016/166316
- KR-A- 20140 100 235
- KR-A- 20190 060 492
- IANNAZZO DANIELA ET AL: "Graphene Quantum Dots Based Systems As HIV Inhibitors", BIOCONJUGATE CHEMISTRY, vol. 29, no. 9, 19 September 2018 (2018-09-19), US, pages 3084 - 3093, XP055888242, ISSN: 1043-1802, Retrieved from the Internet <URL:https://pubs.acs.org/doi/pdf/10.1021/acs.bioconjchem.8b00448> DOI: 10.1021/acs.bioconjchem.8b00448
- WANG JINGYUN ET AL: "Langmuir-Blodgett self-assembly of ultrathin graphene quantum dot films with modulated optical properties", NANOSCALE, vol. 10, no. 41, 1 January 2018 (2018-01-01), United Kingdom, pages 19612 - 19620, XP093048205, ISSN: 2040-3364, DOI: 10.1039/C8NR05159C
- HANJUN SUN ET AL: "Recent advances in graphene quantum dots for sensing", MATERIALS TODAY, vol. 16, no. 11, 1 November 2013 (2013-11-01), AMSTERDAM, NL, pages 433 - 442, XP055417806, ISSN: 1369-7021, DOI: 10.1016/j.mattod.2013.10.020
- DE CLERCQ ERIK ET AL: "Approved antiviral drugs over the past 50 years", CLINICAL MICROBIOLOGY REVIEW, WASHINGTON, DC, US, vol. 29, no. 3, 8 June 2016 (2016-06-08), pages 695 - 747, XP009528410, ISSN: 0893-8512, DOI: 10.1128/CMR.00102-15
- IANNAZZO DANIELA, PISTONE ALESSANDRO, FERRO STEFANIA, DE LUCA LAURA, MONFORTE ANNA MARIA, ROMEO ROBERTO, BUEMI MARIA ROSA, PANNECO: "Graphene Quantum Dots Based Systems As HIV Inhibitors", BIOCONJUGATE CHEMISTRY, AMERICAN CHEMICAL SOCIETY, US, vol. 29, no. 9, 19 September 2018 (2018-09-19), US , pages 3084 - 3093, XP055888242, ISSN: 1043-1802, DOI: 10.1021/acs.bioconjchem.8b00448
- INNOCENZI PLINIO, STAGI LUIGI: "Carbon-based antiviral nanomaterials: graphene, C-dots, and fullerenes. A perspective", CHEMICAL SCIENCE, ROYAL SOCIETY OF CHEMISTRY, UNITED KINGDOM, vol. 11, no. 26, 8 July 2020 (2020-07-08), United Kingdom , pages 6606 - 6622, XP055878064, ISSN: 2041-6520, DOI: 10.1039/D0SC02658A

## Description

### [Technical Field]

The present disclosure relates to an antiviral composition comprising graphene nanoparticles, etc.

This application claims priority from Korean Patent Application Nos. 10-2020-0090617 and 10-2021-0094911, filed on July 21, 2020 and July 20, 2021, respectively.

### [Background Art]

Viruses are obligate microorganisms that proliferate by using host cell metabolism while parasitizing living cells in animals, plants, bacteria, etc. and cause various diseases by destroying or mutating host cells. Viruses are divided into two groups based on the type of nucleic acid: DNA and RNA viruses. Examples of representative viruses that cause diseases harmful to the human body include influenza viruses, herpes viruses, hepatitis B viruses, hepatitis C viruses, human immunodeficiency viruses, papilloma viruses, etc.

Antiviral agents alleviate and treat infectious diseases caused by viruses by weakening the action of viruses that have invaded the human body, and generally act to inhibit the proliferation of viruses and act through the mechanisms of attachment to viruses, inhibition of viral penetration, and inhibition of viral proliferation.

Vaccination is currently the best way to prevent viral diseases, but, in the case of viral diseases, the question of effectiveness of vaccines has been raised as an important issue due to the generation of a great deal of virus serotypes, etc. In the case of the corona virus, which has recently spread widely and is causing serious health threats and social problems worldwide, it is a very serious problem that, although the virus is causing an enormous number of casualties and astronomical economic losses, there are no appropriate preventive measures or cure so far.

Therefore, in order to solve these problems, it is very important to develop and disseminate the technology for preventing viral diseases, and, in particular, it is necessary to develop the technologies for early diagnosis and fundamental blockade of viral transmission.

Meanwhile, graphene nanomaterials are known to have the advantages of high biocompatibility, physical and chemical stability, low toxicity, and excellent dispersibility, and have been mainly used simply as drug delivery systems. Research is being actively conducted on the application of nanomaterials as a new therapeutic agent in life science, but the antiviral effect of graphene nanomaterials has not been suggested.

Iannazzo et al. (Bioconjugate Chemistry, 2018, 29(9):3084-3093) concerns graphene quantum dots for use in the treatment of HIV infections. De Clerck et al. (Clinical microbiology review, 2016, 29(3): 695-747) refers to antiviral agents for use in the treatment of various viral infections, including infections caused by adenovirus.

### [Disclosure]

### [Technical Problem]

The inventors of the present disclosure have completed the present disclosure by demonstrating the excellent antiviral effect of graphene nanomaterials.

Accordingly, the purpose of the present disclosure is to provide an antiviral composition comprising graphene nanoparticles as an active ingredient. The graphene nanoparticles may comprise graphene quantum dots and nano-sized graphene oxide.

Another purpose of the present disclosure is to provide a pharmaceutical composition for preventing or treating viral diseases, comprising the composition according to the present disclosure.

A further purpose of the present disclosure is to provide an antiviral film comprising graphene nanoparticles as an active ingredient.

A further purpose of the present disclosure is to provide an antiviral food composition comprising graphene nanoparticles as an active ingredient.

### [Technical Solution]

Embodiments of the present invention are described in the claims.

According to the present disclosure, there is provided an antiviral composition comprising graphene quantum dots (GQDs) as an active ingredient for use in the treatment of a viral disease, wherein the viral disease is a disease caused by infection with adenovirus.

According to the present disclosure, there is provided a pharmaceutical composition comprising graphene quantum dots (GQDs) as an active ingredient for use in preventing or treating viral diseases, wherein the viral disease is a disease caused by infection with adenovirus.

Furthermore, according to the present disclosure, there is provided an antiviral film comprising graphene quantum dots as an active ingredient for use in preventing or treating a viral disease, wherein the viral disease is a disease caused by infection with adenovirus.

Furthermore, according to the present disclosure, there is provided an antiviral food composition comprising graphene quantum dots as an active ingredient for use in treating a viral disease, wherein the viral disease is a disease caused by infection with adenovirus. The food composition comprises health functional food compositions.

According to an embodiment of the present disclosure, the graphene quantum dots may have an average height of 0.5 to 2.5 nm and an average diameter of 1 to 20 nm.

According to the present disclosure, the composition has antiviral activity against the adenovirus.

According to the present disclosure, the viral disease is a disease caused by infection with adenovirus.

According to another embodiment of the present disclosure, the viral disease may be one or more of pneumonia, otitis media, laryngitis, gastroenteritis, bronchiolitis, meningitis, myocarditis, nephritis, tonsillitis, tonsillitis pharynx, conjunctivitis, pharyngoconjunctival fever, epidemic keratoconjunctivitis, hemorrhagic cystitis, hepatitis, hyperlucent lung syndrome, bronchiectasis, and bronchiolitis obliterans, but is not limited thereto.

Furthermore, according to the present disclosure, there may be provided antiviral health functional food comprising graphene quantum dots as an active ingredient for use in preventing or improving a viral disease, wherein the viral disease is a disease caused by infection with adenovirus.

Furthermore, according to the present disclosure, there may be provided an antiviral feed for use in preventing or improving a viral disease, comprising graphene quantum dots as an active ingredient, wherein the viral disease is a disease caused by infection with adenovirus.

### [Advantageous Effects]

The present disclosure relates to an antiviral composition comprising graphene quantum dots as an active ingredient, and has been completed by confirming that the graphene quantum dots can have an antiviral effect by inhibiting the ability of viruses to infect cells. Therefore, the antiviral composition for use according to the present invention is expected to not only be utilized in various forms, such as a film, a fiber, clothing, food, feed, and a disinfectant, for preventing or blocking viral infections, caused by adenovirus but also be used as an effective therapeutic factor for viral diseases. caused by infection with adenovirus.

### [Description of Drawings]

FIG. 1A is a schematic view of the synthesis of graphene quantum dots according to the present disclosure.
FIG. 1B is a view showing a representative HR-TEM image of graphene quantum dots and the proportion of the graphene quantum dots of each size according to the present disclosure.
FIG. 1C is a view showing a representative AFM image of graphene quantum dots and the distribution of the graphene quantum dots of each height according to the present disclosure.
FIG. 1D shows the results of determining the types of functional groups of the graphene quantum dots according to the present disclosure through the FT-IR (left) and the results of examining the Raman effect through the Raman spectroscopy (right).
FIG. 2 shows the antiviral effect of graphene quantum dots against adeno-virus according to an embodiment of the present disclosure (green: GFP, adeno-virus; blue: DAPI, nucleus).

### [Mode for Disclosure]

According to the present disclosure, there is be provided an antiviral composition comprising graphene quantum dots (GQD) as an active ingredient for use in the treatment of a viral disease, wherein the viral disease is a disease caused by infection with adenovirus.

The term "graphene" used in the present disclosure means that a plurality of carbon atoms are covalently linked to each other to form a polycyclic aromatic molecule. The carbon atoms linked by covalent bonds may form a 6-membered ring as a basic repeating unit, but may also further include a 5-membered ring and/or a 7-membered ring.

The term "nano-sized graphene oxide (nano-GO)" used in the present disclosure refers to a material prepared in the form of nanometer-sized particles by applying a predetermined stimulus to graphene oxide, and the nano-sized graphene oxide may refer to plate-shaped particles having a predetermined height of 12 nm or less and an average diameter of approximately 15 to 50 nm. For example, the nano-sized graphene oxide may be prepared by applying ultrasonic waves to graphene oxide (e.g., by tip-sonification), but is not limited thereto. For example, the nano-sized graphene oxide may have an average diameter of 15 to 45 nm, 15 to 27 nm, 25 to 35 nm, 35 to 45 nm, or 25 to 45 nm. Furthermore, the nanoparticles may be particles having a height of 5 to 12 nm, 3 to 9 nm, 3 to 7 nm, 5 to 9 nm, or 5 to 7 nm, but are not limited thereto.

The term "graphene quantum dots (GQDs)" used in the present disclosure refers to graphene with nano-sized fragments. Specifically, the graphene quantum dots may refer to graphene particles having a width, length, and height of several nanometers, which are prepared through appropriate processing and may be obtained by the thermo-oxidative cutting of carbon fiber, carbon black, graphite, carbon nanotube, etc., but the method of preparing them is not limited thereto.

According to the present disclosure, the graphene quantum dots may be particles having an average height of 0.5 to 2.5 nm and an average diameter of 1 to 20 nm. For example, the graphene quantum dots may have a height of 0.5 to 2 nm, 0.5 to 1.5 nm, 0.5 to 1 nm, 1 to 2.5 nm, 1 to 2 nm, 1 to 1.5 nm, 1.5 to 2.5 nm, 1.5 to 2 nm, or 2 to 2.5 nm, and may have an average diameter of 1 to 20 nm, 1 to 18 nm, 1 to 16 nm, 1 to 14 nm, 1 to 12 nm, 1 to 10 nm, 1 to 9 nm, 1 to 8 nm, and 1 to 7 nm. nm, 1 to 6 nm, 1 to 5 nm, 1 to 4 nm, 1 to 3 nm, 1 to 2 nm, 2 to 15 nm, 2 to 12 nm, 2 to 10 nm, 2 to 9 nm, 2 to 8 nm, 2 to 7 nm, 2 to 6 nm, 2 to 5 nm, 2 to 4 nm, 2 to 3 nm, 3 to 5 nm, 3 to 4 nm, or 4 to 5 nm, but are not limited thereto.

The graphene quantum dots of use in the present disclosure may be in solid or liquid form. Solvates may include non-aqueous solvents such as ethanol, isopropanol, DMSO, acetic acid, ethanolamine, and ethyl acetate, and may include water as a solvent incorporated into a crystalline lattice. Solvates as solvents in which water is incorporated into a crystalline lattice are typically referred to as "hydrates." The present disclosure may include all such solvates.

The "antiviral composition" for use according to the present disclosure may be used without limitation in its form. According to the present disclosure, there may be provided antiviral fiber products comprising the antiviral composition according to the present disclosure for use in accordance with the present disclosure. The antiviral fiber products refer to products whose fibers in the form of yarn or fabric may comprise the antiviral composition of use according to the present disclosure or may be mixed/coated/treated with the same. The antiviral fiber products manufactured using the antiviral composition may have an effect of preventing viral infection and the spread of viruses. There is no limitation in specific examples of the fiber products. However, the examples thereof may include fabrics, clothes, towels, masks, functional clothes, dustproof clothes, protective clothes, surgical clothes, nurse clothes, medical clothes, bed sheets, handkerchiefs, diapers, sanitary pads, sanitary products, medical products, medical devices, bedding, scarves, shoes, car seats, leather products, wet tissues, sheet masks, etc., and may also include a variety of fiber-applied products. The fibers may include polymeric fibers.

In addition, the antiviral composition for use according to the present disclosure may be used for an antiviral disinfectant, disinfection device, spray, soap, ointment, skin lotion, gel, mist, and the like.

According to the present disclosure, the antiviral composition has antiviral activity against adenovirus.

In an embodiment of the present disclosure, the graphene quantum dots were prepared, and their morphological characteristics, functional characteristics, etc. were analyzed (see Embodiment 1).

In another example of the present disclosure, as a result of examining whether the graphene quantum dots have antiviral activity against adenovirus, it was confirmed that the group treated with the graphene quantum dots had a higher level of the antiviral activity than the control group (see Embodiment 2).

Therefore, according to the present disclosure, there is provided a pharmaceutical composition for use in preventing or treating a viral disease, comprising the antiviral composition comprising graphene quantum dots (GQDs) as an active ingredient, wherein the viral disease is a disease caused by infection with adenovirus.

In the present disclosure, the viral disease is a disease caused by infection with adenovirus. More preferably, the viral disease may be one of pneumonia, otitis media, laryngitis, gastroenteritis, bronchiolitis, meningitis, myocarditis, nephritis, tonsillitis, tonsillitis in the throat, conjunctivitis, pharyngoconjunctival fever, epidemic keratoconjunctivitis, hemorrhagic cystitis, hepatitis, hyperlucent lung syndrome, bronchiectasis, bronchiolitis obliterans, etc.

The pharmaceutical composition for use according to the present disclosure may further comprise suitable carriers, excipients, and diluents, which are commonly used in the preparation of pharmaceutical compositions. For example, the excipient may be one or more of a diluent, a binder, a disintegrant, a lubricant, an adsorbent, a moisturizer, a film-coated material, and a controlled release additive.

By conventional methods, the pharmaceutical composition for use according to the present disclosure may be formulated in the form of an external agent such as powders, granules, sustained-release granules, enteric granules, solutions, eye drops, elixirs, emulsions, suspensions, spirits, troches, perfumes, rimonadese, tablets, sustained-release tablets, enteric-coated tablets, sublingual tablets, hard capsules, soft capsules, extended-release capsules, enteric capsules, pills, tinctures, soft extracts, dry extracts, fluid extracts, injections, capsules, perfusate, emplastrum, lotions, pastes, sprays, inhalants, patches, sterile injection solutions, or aerosols. The external agent may have a formulation such as cream, gel, patch, spray, ointment, warning agent, lotion, liniment agent, paste agent, or cataplasma agent.

Examples of the carriers, excipients and diluents that may be contained in the pharmaceutical composition for use according to the present disclosure may include lactose, dextrose, sucrose, oligosaccharides, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

When formulated, it may be prepared with diluents or excipients such as commonly used fillers, extenders, binders, wetting agents, disintegrants, and surfactants.

As an additive for tablets, powders, granules, capsules, pills, and troches according to the present disclosure, an excipient, such as corn starch, potato starch, wheat starch, lactose, white sugar, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methylcellulose, sodium carboxymethylcellulose, kaolin, urea, colloidal silica gel, hydroxypropyl starch, hydroxypropylmethylcellulose (HPMC) 1928, HPMC 2208, HPMC 2906, HPMC 2910, propylene glycol, casein, calcium lactate, and primogel, may be used; a binder, such as gelatin, gum arabic, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, refined shellac, starch, hydroxypropylcellulose, hydroxypropylmethylcellulose, poly vinyl alcohol, and polyvinylpyrrolidone, may be used; a disintegrant, such as hydroxypropyl methyl cellulose, corn starch, agar powder, methyl cellulose, bentonite, hydroxypropyl starch, sodium carboxymethyl cellulose, sodium alginate, calcium carboxymethyl cellulose, calcium citrate, sodium lauryl sulfate, silicic anhydride, 1-hydroxy propyl cellulose, dextran, ion exchange resin, polyvinyl acetate, casein and gelatin treated with formaldehyde, alginic acid, amylose, guar gum, sodium bicarbonate, polyvinylpyrrolidone, calcium phosphate, gelled starch, gum arabic, amylopectin, pectin, sodium polyphosphate, ethyl cellulose, white sugar, magnesium aluminum silicate, di-sorbitol solution, and light anhydrous silicic acid, may be used; and a glydent, such as calcium stearate, magnesium stearate, stearic acid, hydrogenated vegetable oil, talc, lycopod, kaolin, petrolatum, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, macrogol, synthetic aluminum silicate, silicic anhydride, higher fatty acid, higher alcohol, silicone oil, paraffin oil, polyethylene Glycol fatty acid ether, starch, sodium chloride, sodium acetate, sodium oleate, dl-leucine, and light anhydrous silicic acid, may be used.

As an additive for the liquid formulation for use according to the present disclosure, water, dilute hydrochloric acid, dilute sulfuric acid, sodium citrate, sucrose monostearate, polyoxyethylene sorbitol fatty acid esters (twin esters), polyoxyethylene monoalkyl ethers, lanolin ethers, lanolin esters, acetic acid, hydrochloric acid, aqueous ammonia, ammonium carbonate, potassium hydroxide, sodium hydroxide, prolamine, polyvinylpyrrolidone, ethyl cellulose, sodium carboxymethyl cellulose, etc. may be used.

As a syrup according to the present disclosure, a solution of white sugar, other sugars or sweeteners, etc. may be used, and a flavoring agent, a colorant, a preservative, a stabilizer, a suspending agent, an emulsifier, a viscous agent, etc. may be used as needed.

Purified water may be used as an emulsion according to the present disclosure, and an emulsifier, a preservative, a stabilizer, a flavoring agent, etc. may be used as needed.

As a suspending agent according to the present disclosure, a suspending agent such as acacia, tragacanth, methyl cellulose, carboxymethyl cellulose, sodium carboxymethyl cellulose, microcrystalline cellulose, sodium alginate, HPMC 1828, HPMC 2906, and HPMC 2910 may be used, and a surfactant, a preservative, a stabilizer, a colorant, and a flavoring agent may be used as needed.

Examples of the injection according to the present disclosure may include a solvent, such as distilled water for injection, 0.9 % sodium chloride injection, IV injection, dextrose injection, dextrose + sodium chloride injection, PEG, lactated IV injection, ethanol, propylene glycol, non-volatile oil - sesame oil, cottonseed oil, peanut oil, Soybean oil, corn oil, ethyl oleate, isopropyl myristate, and benzene benzoate; a solubilizing agent such as sodium benzoate, sodium salicylate, sodium acetate, urea, urethane, monoethylacetamide, butazolidine, propylene glycol, twins, nijuntinamide, hexamine, and dimethylacetamide; a buffer such as weak acids and their salts (acetic acid and sodium acetate), weak bases and their salts (ammonia and ammonium acetate), organic compounds, proteins, albumin, peptone, and gums; a tonicity agent such as sodium chloride; a stabilizer such as sodium bisulfite (NaHSO₃) carbon dioxide gas, sodium metabisulfite (Na₂S₂O₃), sodium sulfite (Na₂SO₃), nitrogen gas (N₂), and ethylenediamine tetraacetic acid; a sulfating agent such as sodium bisulfide 0.1 %, sodium formaldehyde sulfoxylate, thiourea, ethylenediamine disodium tetraacetate, and acetone sodium bisulfite; an analgesic such as benzyl alcohol, chlorobutanol, procaine hydrochloride, glucose, and calcium gluconate; and a suspending agent such as SiMCsodium, sodium alginate, Tween 80, and aluminum monostearate.

As a suppository according to the present disclosure, a base such as cacao butter, lanolin, witapsol, polyethylene glycol, glycerogelatin, methyl cellulose, carboxymethyl cellulose, mixture of stearic acid and oleic acid, subanal, cottonseed oil, peanut oil, palm oil, cacao butter + cholesterol, lecithin, lanet wax, glycerol monostearate, tween or span, imhausen, monolen (propylene glycol monostearate), glycerin, adeps solidus, Buytyrum Tego-G, cebes pharma 16, hexalide base 95, cotomar, hydrokote SP, S-70-XXA, S-70-XX75 (S-70-XX95), hydrokote 25, hydrokote 711, idropostal, massa estrarium (A, AS, B, C, D, E, I, T), martha-MF, marsupol, marsupol-15, neosupostal-N, paramound-B, suposiro (OSI, OSIX, A, B, C, D, H, L), suppository type IV (AB , B, A, BC, BBG, E, BGF, C, D, 299), supostal (N, Es), wecobi (W, R, S, M, Fs), and testester triglyceride base (TG- 95, MA, 57) may be used.

Examples of solid formulations for oral administration may include tablets, pills, powders, granules, capsules, etc., and these solid formulations may be prepared by mixing the extract with at least one excipient such as starch, calcium carbonate, sucrose or lactose, and gelatin. In addition to simple excipients, lubricants such as magnesium stearate and talc may also be used.

Examples of liquid formulations for oral administration may include suspensions, internal solutions, emulsions, syrups, etc., and various excipients such as wetting agents, sweeteners, flavoring agents, and preservatives may be included in addition to water and liquid paraffin, which are commonly used simple diluents. Examples of formulations for parenteral administration may include sterilized aqueous solutions, non-aqueous solvents, suspensions, emulsions, freeze-dried formulations, and suppositories. Propylene glycol, polyethylene glycol, vegetable oils such as olive oil, injectable esters such as ethyl oleate, etc. may be used as non-aqueous solvents and suspending agents.

The pharmaceutical composition for use according to the present disclosure may be administered in a pharmaceutically effective amount. In the present disclosure, a "pharmaceutically effective amount" means an amount sufficient to treat a disease with a reasonable benefit / risk ratio applicable to medical treatment, and the effective amount may be determined according to the type of disease, the severity of disease, the activity of drug, the sensitivity to drug, the time of administration, the route of administration and the excretion rate, the duration of treatment, factors including drugs used concurrently, and other factors well known in the medical field.

The pharmaceutical composition for use according to the present disclosure may be administered as an individual therapeutic agent or in combination with other therapeutic agents, and may be administered sequentially or concurrently with an existing therapeutic agent and once or multiple times. It is important to administer the pharmaceutical composition for use according to the present disclosure in an amount to obtain the maximum effect with the minimum amount without side effects by considering all the above-mentioned factors, and such amount may be easily determined by a person having ordinary skill in the technical field to which the present disclosure pertains.

The pharmaceutical composition for use according to the present disclosure may be administered to a subject by various routes. All methods of administration may be expectable and may include oral administration, subcutaneous injection, intraperitoneal administration, intravenous injection, intramuscular injection, injection in the paraspinal space (intrathecal), sublingual administration, buccal administration, insertion into the rectum, insertion into the vagina, intraocular administration, ear administration, nasal administration, inhalation, spraying through the mouth or nose, dermal administration, transdermal administration, etc.

The pharmaceutical composition for use according to the present disclosure may be determined according to the type of drug as an active ingredient together with various related factors such as the type of disease to be treated, the route of administration, the patient's age, the patient's gender, the patient's weight, and the severity of disease.

In the present disclosure, a "subject" means a subject in need of treatment of a disease, and, more specifically, a mammal such as a human or non-human primate, mouse, rat, dog, cat, horse, and cow.

In the present disclosure, "administering" means providing the predetermined composition according to the present disclosure to a subject by any suitable method.

In the present disclosure, "prevention" refers to all actions to suppress or delay the onset of a target disease, "treatment" refers to all actions to improve or beneficially change the target disease and the resulting metabolic abnormality through the administration of the pharmaceutical composition according to the present disclosure, and "improvement" refers to all actions to reduce a parameter related to the target disease, e.g., the severity of a symptom, by administration of the composition according to the present disclosure.

Furthermore, in the present disclosure, the antiviral composition may be a cosmetic composition comprising graphene quantum dots (GQDs) as an active ingredient for use in preventing or improving a viral disease, wherein the viral disease is a disease caused by infection with adenovirus.

Examples of the formulation of the cosmetic composition for use according to the present disclosure may include skin lotion, skin softener, skin toner, astringent, lotion, milk lotion, moisture lotion, nutrient lotion, massage cream, nutrient cream, mist, moisture cream, hand cream, hand lotion, foundation, essence, nutritional essence, pack, soap, cleansing foam, cleansing lotion, cleansing cream, cleansing oil, cleansing balm, body lotion, and body cleanser.

The cosmetic composition for use according to the present disclosure may further include a composition selected from the group consisting of water-soluble vitamins, oil-soluble vitamins, high-molecular peptides, high-molecular polysaccharides, and sphingolipids.

Any water-soluble vitamin may be used as long as it can be formulated into cosmetics, but vitamin B 1, vitamin B2, vitamin B6, pyridoxine, pyridoxine hydrochloride, vitamin B 12, pantothenic acid, nicotinic acid, nicotinic acid amide, folic acid, vitamin C, vitamin H, etc. may be used. In addition, their salts (thiamine hydrochloride, sodium ascorbate, etc.) or derivatives (sodium ascorbic acid-2-phosphate, magnesium ascorbic acid-2-phosphate, etc.) may also be included in the water-soluble vitamins used in the present disclosure. The water-soluble vitamins may be obtained by conventional methods such as microbial transformation, purification from microbial culture, and enzymatic or chemical synthesis.

Any oil-soluble vitamin may be used as long as it can be formulated into cosmetics, but vitamin A, carotene, vitamin D2, vitamin D3, vitamin E (d1-alpha tocopherol, d-alpha tocopherol, and d-alpha tocopherol), etc. may be used. In addition, their derivatives (ascorbine palmitate, ascorbine stearate, ascorbine dipalmitate, dl-alpha tocopherol acetate, dl-alpha tocopherol nicotinate, vitamin E, DL-pantothenyl alcohol, D-pantothenyl alcohol, pantothenyl ethyl ether, etc.) may also be included in the oil-soluble vitamins used in the present disclosure. The oil-soluble vitamins may be obtained by conventional methods such as microbial transformation, purification from microbial culture, and enzymatic or chemical synthesis.

Any polymer peptide may be used as long as it can be formulated into cosmetics, but collagen, hydrolyzed collagen, gelatin, elastin, hydrolyzed elastin, keratin, etc. may be used. The polymeric peptides may be purified and obtained by conventional methods such as purification from a culture medium of microorganisms and enzyme or chemical synthesis, and may be used after being purified from natural products such as dermis of pigs, cows, etc., and silk fibers of silkworms.

Any polymeric polysaccharide may be used as long as it can be formulated into cosmetics, but hydroxyethyl cellulose, xanthan gum, sodium hyaluronate, chondroitin sulfate or its salts (sodium salt, etc.), and the like may be used. For example, chondroitin sulfate or its salts may be used after being purified from mammals or fish.

Any sphingolipid may be used as long as it can be formulated into cosmetics, but ceramide, phytosphingosine, sphingoglycolipid, etc. may be used. The sphingolipids may be purified by conventional methods or obtained by chemical synthesis from mammals, fish, shellfish, yeast, plants, etc.

The cosmetic composition for use according to the present disclosure may be combined with other components commonly formulated in cosmetics in addition to the above-mentioned essential components, if necessary.

Examples of other blending components that can be added may include oil components, humectants, emollients, surfactants, organic and inorganic pigments, organic powders, ultraviolet absorbers, preservatives, bactericides, antioxidants, plant extracts, pH adjusters, alcohols, pigments, fragrances, blood circulation promoters, cooling agents, antiperspirants, purified water, etc.

Examples of the oil component may include ester oils, hydrocarbon oils, silicone oils, fluorine oils, animal fats, vegetable oils, etc.

Examples of the ester oil may include esters such as glyceryl tri-2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linoleate , isopropyl linoleate, ethyl oleate, isocetyl myristate, isostearyl myristate, isostearyl palmitate, octyldodecyl myristate, isocetyl isostearate, diethyl sebacate, diisopropyl adipate, isoalkyl neopentanoate , tri (capryl, capric acid) glyceryl, isocetyl palmitate, octyl stearate, isocetyl stearate, isodecyl oleate, octyldodecyl oleate, linoleic acid octyldodecyl, isopropyl isostearate, 2-ethylhexanoate cetostearyl, 2-ethylhexanoate stearyl, isostearate hexyl, ethylene glycol dioctanoate, ethylene glycol dioleate, propylene glycol dicaprate, di (caprylic, capric acid) propylene glycol, propylene glycol dicaprylate, neopentyl glycol dicaprate, neopentyl glycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin oleate, polyglycerin isostearate, triisocetyl citrate, triisoalkyl citrate, triisooctyl citrate, lauryl lactate, myristyl lactate, cetyl lactate , octyldecyl lactate, triethyl citrate, acetyl triethyl citrate, acetyl tributyl citrate, trioctyl citrate, diisostearyl malate, 2-ethylhexyl hydroxystearate, di2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phyteryl isostearate, physteryl oleate, isocetyl 12-stealoylhydroxystearate, stearyl 12-stealoylhydroxystearate, and 12-stearoyl hydroxy stearate isostearyl.

Examples of the hydrocarbon oil may include squalene, liquid paraffin, alpha-olefin oligomer, isoparaffin, ceresin, paraffin, liquid isoparaffin, polybuden, microcrystalline wax, Vaseline, etc.

Examples of the silicone oil may include polymethylsilicone, methylphenylsilicone, methylcyclopolysiloxane, octamethylpolysiloxane, decamethylpolysiloxane, dodecamethylcyclosiloxane, dimethylsiloxane/methylcetyloxysiloxane copolymer, dimethylsiloxane/methylstealoxysiloxane copolymer, alkyl modified silicone oil, amino modified silicone oil, etc.

Examples of the fluorine oil may include perfluoropolyether and the like.

Examples of animal or vegetable oil may include avocado oil, almond oil, olive oil, sesame oil, rice bran oil, safflower oil, soybean oil, corn oil, rapeseed oil, algae oil, palm kernel oil, palm oil, castor oil, sunflower oil, grape seed oil, cottonseed oil, palm oil, kukui nut oil, wheat germ oil, rice germ oil, shea butter, moonshine colostrum, macadamia nut oil, meadowfoam oil, egg yolk oil, beef tallow, horse oil, mink oil, orange raffia oil, jojoba oil, candelilla wax, carnaba wax, liquid lanolin, hydrogenated castor oil, etc.

Examples of the moisturizer may include water-soluble low-molecular moisturizers, oil-soluble low-molecular moisturizers, water-soluble polymers, oil-soluble polymers, etc.

Examples of the water-soluble low-molecular moisturizer may include serine, glutamine, sorbitol, mannitol, pyrrolidone-sodium carboxylate, glycerin, propylene glycol, 1,3-butylene glycol, ethylene glycol, polyethylene glycol B (polymerization degree n = 2 or more), polypropylene glycol (polymerization degree n = 2 or more), polyglycerol B (polymerization degree n = 2 or more), lactic acid, lactate, etc.

Examples of the oil-soluble low-molecular moisturizer may include cholesterol, cholesterol ester, etc.

Examples of the water-soluble polymer may include carboxyvinyl polymer, polyaspartate, tragacanth, xanthan gum, methyl cellulose, hydroxymethyl cellulose, hydroxyethyl cellulose, hydroxypropyl cellulose, carboxymethyl cellulose, water-soluble chitin, chitosan, dextrin, and the like.

Examples of the oil-soluble polymer may include polyvinylpyrrolidone/eicosene copolymer, polyvinylpyrrolidone/hexadecene copolymer, nitrocellulose, dextrin fatty acid ester, high molecular silicone, etc.

Examples of the emollient agent may include long-chain acyl glutamic acid cholesteryl ester, hydroxystearic acid cholesteryl, 12-hydroxystearic acid, stearic acid, rosin acid, lanolin fatty acid cholesteryl ester, etc.

Examples of the surfactant may include nonionic surfactants, anionic surfactants, cationic surfactants, amphoteric surfactants, etc.

Examples of the nonionic surfactant may include self-emulsifying glycerin monostearate, propylene glycol fatty acid ester, glycerin fatty acid ester, polyglycerin fatty acid ester, sorbitan fatty acid ester, polyoxyethylene (POE), sorbitan fatty acid ester, POE sorbitan fatty acid ester, POE glycerin fatty acid ester, POE alkyl ether, POE fatty acid ester, POE hydrogenated castor oil, POE castor oil, polyoxyethylene/polyoxypropylene (POE/POP) copolymer, POE/POP alkyl ether, polyether modified silicone, lauric acid alkanolamide, alkylamine oxide, hydrogenated soybean phospholipids, etc.

Examples of the anionic surfactant may include fatty acid soaps, alpha-acyl sulfonates, alkyl sulfonates, alkyl allyl sulfonates, alkyl naphthalene sulfonates, alkyl sulfates, POE alkyl ether sulfates, alkyl amide sulfates, alkyl phosphates, POE alkyl phosphates, alkyl amide phosphates, alkyloylalkyl taurine salt, N-acylamino acid salt, POE alkyl ether carboxylate, alkyl sulfosuccinate, sodium alkyl sulfoacetate, acylated hydrolyzed collagen peptide salt, perfluoroalkyl phosphate ester, etc.

Examples of the cationic surfactant may include alkyltrimethylammonium chloride, stearyltrimethylammonium chloride, stearyltrimethylammonium bromide, cetostearyltrimethylammonium chloride, distearyldimethylammonium chloride, stearyldimethylbenzylammonium chloride, behenyltrimethylammonium bromide, benzalkonium chloride, diethylaminoethyl stearate, stearic acid dimethylaminopropylamide, lanolin derivative quaternary ammonium salt, etc.

Examples of the amphoteric surfactant may include carboxybetaine type, amidebetaine type, sulfobetaine type, hydroxysulfobetaine type, amidesulphobetaine type, phosphobetaine type, aminocarboxylate type, imidazoline derivative type, amideamine type, etc.

Examples of the organic and inorganic pigment may include an inorganic pigment such as silicic acid, silicic anhydride, magnesium silicate, talc, sericite, mica, kaolin, bengala, clay, bentonite, titanium-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and complexes thereof; an organic pigment such as polyamide, polyester, polypropylene, polystyrene, polyurethane, vinyl resin, urea resin, phenolic resin, fluororesin, silicon resin, acrylic resin, melamine resin, epoxy resin, polycarbonate resin, divinylbenzene/styrene copolymer, silk powder, cellulose, CI pigment yellow, and CI pigment orange; complexes of these inorganic and organic pigments; and so on.

Examples of the organic powder may include metal soaps such as calcium stearate; alkyl phosphate metal salts such as zinc sodium cetylrate, zinc laurate, and calcium laurate; polyvalent metal salts of acyl amino acids such as N-lauroyl-beta-alanine calcium, N-lauroyl-beta-alanine zinc, and N-lauroyl glycine calcium; polyvalent metal salts of amide sulfonic acids such as N-lauroyl-taurine calcium and N-palmitoyl-taurine calcium; N-acyl basic amino acid such as N-epsilon-lauroyl-L-lysine, N-epsilon-palmitoylizine, N-alpha-paritoylolnithine, N-alpha-lauroylarginine, and N-alpha-hydrogenated beef fatty acid acylarginine; N-acyl polypeptides such as N-lauroylglycylglycine; alpha-amino fatty acids such as alpha-aminocaprylic acid and alpha-aminolauric acid; polyethylene; polypropylene; nylon; polymethyl methacrylate; polystyrene; divinylbenzene/styrene copolymer; tetrafluoroethylene; etc.

Examples of the UV absorber may include para-aminobenzoic acid, ethyl para-aminobenzoate, amyl para-aminobenzoate, octyl para-aminobenzoate, ethylene glycol salicylate, phenyl salicylate, octyl salicylate, benzyl salicylate, butylphenyl salicylate, homomenthyl salicylate, benzyl cinnamate, para-methoxycinnamic acid-2-ethoxyethyl, octyl paramethoxycinnamic acid, diparamethoxycinnamic acid mono-2-ethylhexane glyceryl, isopropyl paramethoxycinnamic acid, a mixture of diisopropyl and diisopropylcinnamic acid ester, urocanic acid, ethyl urocaninate, hydroxymethoxy benzophenone, hydroxymethoxybenzophenonesulfonic acid and salts thereof, dihydroxymethoxybenzophenone, dihydroxymethoxybenzophenone disulfonate sodium, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, etc.

Examples of the disinfectant may include hinokitiol, triclosan, trichlorohydroxydiphenyl ether, chlorhexidine gluconate, phenoxyethanol, resorcin, isopropylmethylphenol, azulene, salicylic acid, zincphyllione, benzalkonium chloride, photosensitizer No. 301, mononitroguacol sodium, undecyrenic acid, etc.

Examples of the antioxidant may include butylhydroxyanisole, propyl gallic acid, elisorbic acid, etc.

Examples of the pH adjuster may include citric acid, sodium citrate, malic acid, sodium malate, fumaric acid, sodium fumalate, succinic acid, sodium succinate, sodium hydroxide, sodium monohydrogen phosphate, etc.

Examples of the alcohol may include higher alcohols such as cetyl alcohol.

In addition, other blending components that can be added are not limited thereto. Any of the above-mentioned components can be blended as long as it does not impair the purposes and effects of the present disclosure, but may be blended at 0.01 to 5 % or 0.01 to 3 % weight percentage relative to the total weight.

When the formulation according to the present disclosure is a lotion, paste, cream, or gel, animal fibers, vegetable fibers, wax, paraffin, starch, tracanth, cellulose derivatives, polyethylene glycol, silicone, bentonite, silica, talc, zinc oxide, etc. may be used as carrier components.

When the formulation according to the present disclosure is a powder or spray, lactose, talc, silica, aluminum hydroxide, calcium silicate, or polyamide powders may be used as carrier components. In particular, when the formulation is a spray, propellants such as chlorofluorohydrocarbon, propane/butane, or dimethyl ether may additionally be used.

When the formulation according to the present disclosure is a solution or emulsion, as carrier components, a solvent, solvating agent, or emulsifying agent, such as water, ethanol, isopropanol, ethyl carbonate, ethyl acetate, benzyl alcohol, benzyl benzoate, propylene glycol, 1,3-butylglycol oil, glycerol aliphatic esters, polyethylene glycol, or fatty acid esters of sorbitan, may be used.

When the formulation according to the present disclosure is a suspension, as carrier components, liquid diluents such as water, ethanol, or propylene glycol; suspending agents such as ethoxylated isostearyl alcohols, polyoxyethylene sorbitol esters, and polyoxyethylene sorbitan esters; microcrystalline cellulose; aluminum metahydroxide; bentonite; agar; tracanth; etc. may be used.

When the formulation according to the present disclosure is a cleanser with surfactants, as carrier components, fatty alcohol sulfates, fatty alcohol ether sulfates, sulfosuccinic acid monoesters, isethionates, imidazolinium derivatives, methyl taurates, sarcosinates, fatty acid amide ether sulfates, alkylamidobetaines, fatty alcohols, fatty acid glycerides, fatty acids diethanolamide, vegetable oil, linolin derivatives, ethoxylated glycerol fatty acid esters, etc. may be used.

Furthermore, the antiviral composition for use according to the present disclosure may be an antiviral food composition or feed composition. The antiviral composition may be a food composition or feed composition comprising graphene quantum dots (GQDs) as an active ingredient for use in preventing or improving a viral disease, wherein the viral disease is a disease caused by infection with adenovirus.

When the graphene quantum dots of use in the present disclosure are used as a food additive, they may be added as they are or used together with other foods or food ingredients, and may be appropriately used in a conventional method. The mixing amount of active ingredients may be appropriately determined according to the purpose of use (e.g., prevention, health care, or therapeutic treatment). In general, when preparing food or beverages, the graphene quantum dots of use in the present disclosure may be added in an amount of 15 % by weight or less or 10 % by weight or less based on the raw material. However, the amount may be reduced when consumed for a long period of time for the purpose of health care and hygiene or health condition control, and active ingredients may be used in an amount equal to or larger than the above-mentioned amount since there is no problem in terms of safety.

There is no particular limitation in the type of the foods. Examples of foods to which the substances can be added may include meat, sausage, bread, chocolate, candy, snacks, confectionery, pizza, ramen, other noodles, gum, dairy products including ice cream, various soups, beverages, tea, drinks, alcoholic beverages, vitamin complexes, etc., and may include all of health functional foods in the usual sense.

The health beverage composition for use according to the present disclosure may contain various flavoring agents, natural carbohydrates, etc. as additional components, as do conventional beverages. The aforementioned natural carbohydrates may be monosaccharides such as glucose and fructose, disaccharides such as maltose and sucrose, polysaccharides such as dextrins and cyclodextrins, and sugar alcohols such as xylitol, sorbitol, and erythritol. As sweeteners, natural sweeteners such as thaumatin and stevia extracts, synthetic sweeteners such as saccharin and aspartame, etc. may be used. The proportion of the natural carbohydrates per 100 mL of the composition according to the present disclosure may be generally approximately 0.01 to 0.20 g or 0.04 to 0.10 g.

In addition to the above-mentioned substances, the composition for use according to the present disclosure may contain various nutrients, vitamins, electrolytes, flavors, colorants, pectic acid and its salts, alginic acid and its salts, organic acids, protective colloidal thickeners, pH adjusters, stabilizers, preservatives, glycerin, alcohol, carbonating agents used for carbonated beverages, and the like. Furthermore, the composition according to the present disclosure may contain fruit flesh for preparing natural fruit juice, fruit juice beverages, and vegetable beverages. These components may be used individually or in combination. The proportion of these additives may not be critical, but may generally be within the range of 0.01 to 0.20 parts by weight per 100 parts by weight of the composition according to the present disclosure.

The term "feed" used in the preset disclosure refers to any natural or artificial diet, meal, etc. or components in the meal, for or suitable for eating, ingesting, and digesting by an animal. The feed containing graphene quantum dots (GQDs) as an active ingredient for use according to the present disclosure may be prepared as various types of feed known in the industry, and examples thereof may specifically include concentrated feed, roughage and/or special feed.

The graphene quantum dots (GQDs) of use in the present disclosure included in the feed composition for use according to the present disclosure may vary depending on the purpose and conditions of use of feed, and may be included in 0.01 to 100 % by weight, more specifically, 1 to 80 % by weight, based on the total weight of a livestock feed composition, for example. However, they are not limited thereto.

According to another aspect of the present disclosure, there is provided an antiviral film comprising graphene quantum dots as an active ingredient for use in preventing or treating a viral disease, wherein the viral disease is a disease caused by infection with adenovirus.

The antiviral film may be prepared by coating, mixing, and treating polymer material films such as polyethylene terephthalate (PET), polypropylene (PP), and polyethylene (PE) with graphene nanoparticles, and by inserting, spraying, and storing the graphene nanoparticles into the polymer material films, but is not limited thereto. The film may be an adhesive film or a coating film. Therefore, the antiviral film according to the present disclosure may be applied to components of public facilities used by a number of people, e.g., parts that many people's bodies touch such as doors or their handles installed for entrance or exit, handles of public transportation facilities, handles of transport carts used for shopping, and elevator buttons, thereby maintaining hygiene and cleanliness by sterilizing and removing various pathogens.

Hereinafter, desirable embodiments and experimental examples will be presented to aid understanding of the present disclosure. However, the following embodiments and experimental examples are only provided to make the present disclosure easier to understand, and the content of the present disclosure is not limited by the embodiments and experimental examples.

### Embodiment 1. Preparation of Graphene Quantum Dots (GQDs)

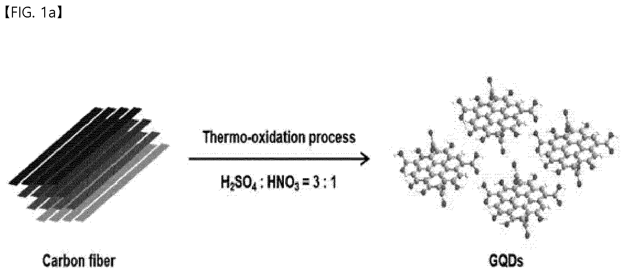

Carbon fibers were placed in a strong acid solution in which sulfuric acid and nitric acid had been mixed in a ratio of 3:1, and then heated at 80 °C for 24 hours (thermo-oxidation process). In order to prepare graphene quantum dots in powder form, the solution was diluted, dialyzed against a regenerated nitrocellulose membrane (Cat#: 06-680-2G; MWCO 1,000 daltons; Fisher Scientific) to remove acid, vacuum-filtered through a porous inorganic membrane filter (Cat#: 6809-5002; Whatman-Anodisc 47; GE Healthcare), and freeze-dried. The graphene quantum dot powder was redispersed in distilled water to be used in the experiment (FIG. 1A).

### Embodiment 2. Analysis of Characteristics of Graphene Quantum Dots

In order to measure the average shape of the graphene quantum dots, the diameter (lateral size) of the graphene quantum dots was measured by high-resolution transmission electron microscopy (HR-TEM), and the height thereof was measured by atomic force microscopy (AFM). In addition, the Fourier-transform infrared spectroscopy (FT-IR) and the Raman spectroscopy were performed to determine the types of functional groups of the graphene quantum dots.

Specifically, before using a HR-TEM, a 300-mesh copper TEM grid with lacey carbon (01890-F, TedPella, USA) was coated with graphene. The diluted graphene quantum dot solution was dropped onto the grid and dried in air. The images of the graphene quantum dots were obtained by a HR-TEM (JEM-3010, JEOL Ltd, Japan) and Gatan digital camera (MSC-794). The diameter of the graphene quantum dots was measured by the Image J.

For the AFM, a graphene quantum dot solution was dropped onto a 1×1 Si wafer and dried to prepare a sample, and it was analyzed in a contact mode using an AFM (Park Systems, Suwon, Korea).

For the FT-IR, a graphene quantum dot sample was prepared by a conventional KBr pellet method, and the spectrum was measured with an FT-IR spectrometer (Nicolet 6700, Thermo Scientific, USA). Prior to the measurement, the powdered sample was completely dried in a desiccator to exclude unwanted oxygen containing peaks. The spectrum was measured by a conventional KBR pellet method (Nicolet 6700, Thermo Scientific).

For the Raman effect, a sample was prepared by drying a graphene quantum dot solution on a 1×1 Si wafer, and the spectrum was measured using a Raman spectrometer (Micro-Raman spectrometer, Renishaw, UK) with a 514 nm laser.

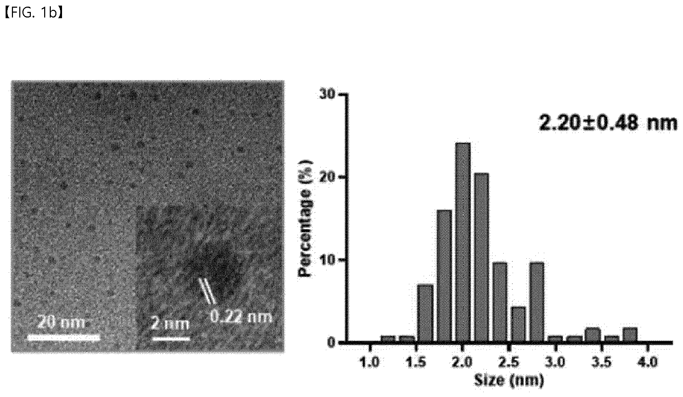

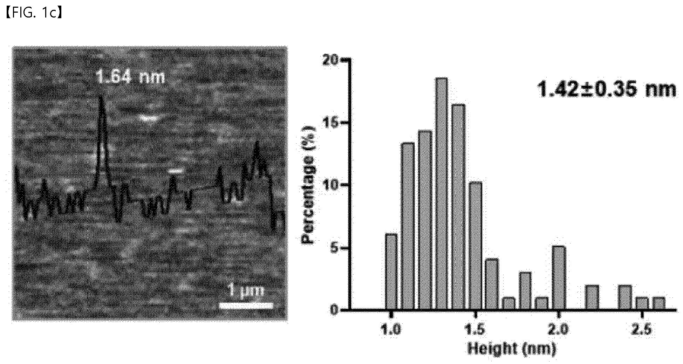

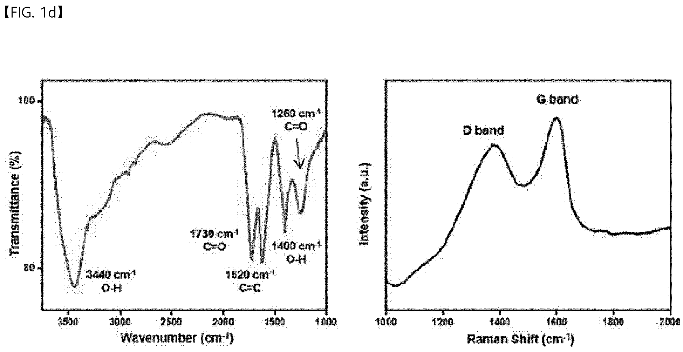

As a result, it was found that the graphene quantum dots had a diameter of 2.20 nm (FIG. 1B) and a height of 1.42 nm (FIG. 1C). The wave numbers corresponding to the stretching of the carboxyl group (C=O) and hydroxyl group (O-H) in the edge area appeared at 1730 cm⁻¹ and 3440 cm⁻¹, respectively, and the wave number corresponding to the stretching of sp² C=C in the graphene area appeared at 1620 cm⁻¹ (the left figure of FIG. 1D). As a result of analyzing the Raman shift, a D band (1400 cm⁻¹) and a G band (1600 cm⁻¹) of graphene were observed (the right figure of FIG. 1D).

### Embodiment 3. Antiviral Effect of Graphene Quantum Dots

In order to confirm the antiviral effect of graphene quantum dots, after graphene quantum dots at a concentration of 1 ug/ml and adeno-virus were cultured for 30 minutes, primary cultured cortical-neurons obtained from mice were treated with the cultured graphene quantum dots and adenovirus for seven days. Then, by an immunofluorescence (IF) analysis, the level of adenovirus infection of the brain neurons was measured.

Specifically, to perform the immunofluorescence analysis, cells were fixed in 4 % paraformaldehyde (PFA) PBS solution at room temperature for 15 minutes and treated with 0.25 % Triton X-100 (Sigma) for 10 minutes to be more permeable. The fixed cells were incubated with a blocking solution (5 % normal goat serum) for one hour at room temperature and incubated with primary antibody overnight at 4 °C. The cells were then incubated with a secondary antibody labeled with Alexa Fluor 594 (Invitrogen), followed by the DAPI (Sigma) staining for five minutes to stain the nuclei. Viral proteins were labeled with GFP, and the images were obtained by a confocal microscope (Eclipse TE200, Nikon, Japan).

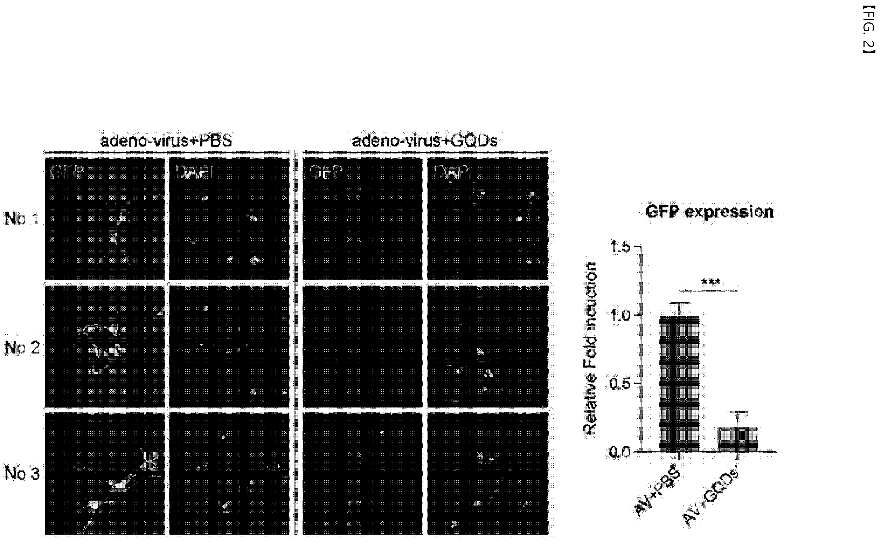

As a result, as shown in FIG. 2, it was found that cells treated with viruses cultured with graphene quantum dots had a significantly lower level of virus infection by approximately 70 % or more compared to the control group (treated with viruses not cultured with graphene quantum dots). The results indicate that the graphene quantum dots according to the present disclosure may have antiviral activity and be capable of inhibiting the infectivity of viruses.

### [industrial Applicability]

The present disclosure relates to an antiviral composition comprising graphene quantum dots as an active ingredient for use in the treatment of a viral disease wherein the viral disease is a disease caused by infection with adenovirus, and has been completed by confirming that the graphene quantum dots can have an antiviral effect by inhibiting the ability of viruses to infect cells. Therefore, the antiviral composition for use according to the present invention is expected to not only be utilized in various forms, such as a film, a fiber, clothing, food, feed, and a disinfectant, for preventing or blocking viral infections, but also be used as an effective therapeutic factor for viral diseases.

## Claims

1. A composition comprising graphene quantum dots (GQDs) as an active ingredient for use in the treatment of a viral disease, wherein the viral disease is a disease caused by infection with adenovirus.

2. The composition for use of claim 1, wherein the graphene quantum dots have an average height of 0.5 to 2.5 nm and an average diameter of 1 to 20 nm.

3. The composition for use of claim 1 or claim 2, wherein the composition is an antiviral composition, a pharmaceutical composition, or a food composition.

4. An antiviral film comprising graphene quantum dots as an active ingredient for use in preventing or treating a viral disease, wherein the viral disease is a disease caused by infection with adenovirus.

## Patentansprüche

1. Zusammensetzung, die Graphen-Quantenpunkte (GQDs) als einen Wirkstoff zur Verwendung bei der Behandlung einer Viruserkrankung umfasst, wobei die Viruserkrankung eine Erkrankung ist, die durch eine Infektion mit Adenovirus verursacht wird.

2. Zusammensetzung zur Verwendung nach Anspruch 1, wobei die Graphen-Quantenpunkte eine durchschnittliche Höhe von 0,5 bis 2,5 nm und einen durchschnittlichen Durchmesser von 1 bis 20 nm aufweisen.

3. Zusammensetzung zur Verwendung nach Anspruch 1 oder Anspruch 2, wobei die Zusammensetzung eine antivirale Zusammensetzung, eine pharmazeutische Zusammensetzung oder eine Lebensmittelzusammensetzung ist.

4. Antiviraler Film, der Graphen-Quantenpunkte als Wirkstoff umfasst, zur Verwendung bei der Vorbeugung oder Behandlung einer Viruserkrankung, wobei die Viruserkrankung eine Erkrankung ist, die durch eine Infektion mit Adenovirus verursacht wird.

## Revendications

1. Composition comprenant des points quantiques de graphène (GQD) comme principe actif, destinée à être utilisée dans le traitement d'une maladie virale, dans laquelle la maladie virale est une maladie causée par une infection à adénovirus.

2. Composition destinée à être utilisée selon la revendication 1, dans laquelle les points quantiques de graphène présentent une hauteur moyenne de 0,5 à 2,5 nm et un diamètre moyen de 1 à 20 nm.

3. Composition destinée à être utilisée selon la revendication 1 ou la revendication 2, dans laquelle la composition est une composition antivirale, une composition pharmaceutique ou une composition alimentaire.

4. Film antiviral comprenant des points quantiques de graphène comme principe actif, destiné à être utilisé dans la prévention ou le traitement d'une maladie virale, dans lequel la maladie virale est une maladie causée par une infection à adénovirus.
